# FASCICULE DE BREVET EUROPEEN

(11) **EP 2 825 631 B1**
(45) Date de publication et mention de la délivrance du brevet: **06.09.2017**
(21) Numéro de dépôt: 13715330.0
(22) Date de dépôt: 15.03.2013
(51) Int. Cl.: C12N 1/12, C12N 13/00, C12P 7/64, C12P 23/00

(54) **PRODUCTION D'ACIDE DOCOSAHEXAÉNOÏQUE ET D'ASTAXANTHINE EN MODE MIXOTROPHE PAR SCHIZOCHYTRIUM.**
HERSTELLUNG VON ASTAXANTHIN UND DOCOSAHEXAENSÄURE IN EINEM MIXOTROPHEN MODUS MITHILFE VON SCHIZOCHYTRIUM
PRODUCTION OF ASTAXANTHIN AND DOCOSAHEXAENOIC ACID IN MIXOTROPHIC MODE USING SCHIZOCHYTRIUM

(30) Priorité: 16.03.2012 FR 1252380
(43) Date de publication de la demande: 21.01.2015
(73) Titulaire: Fermentalg, 33500 Libourne (FR)
(72) Inventeur: ROMARI, Khadidja, 34200 Sète (FR); LE MONNIER, Adeline, 56450 Surzur (FR); ROLS, Cyril, 13004 Marseille (FR); MERLET, Cindy, 33910 Saint Denis De Pile (FR); PAGLIARDINI, Julien, 33150 Cenon (FR); CALLEJA, Pierre, 33500 Libourne (FR); GUDIN, Claude, 13100 Aix En Provence (FR)
(74) Mandataire: Be IP
(86) Numéro de dépôt international: PCT/FR2013/050547
(87) Numéro de publication internationale: WO 2013/136028

(56) Documents cités:
- WO-A1-96/21723
- WO-A1-2009/134114
- CHATDUMRONG W ET AL: "Optimization of docosahexaenoic acid (DHA) production and improvement of astaxanthin content in a mutant Schizochytrium limacinum isolated from mangrove forest in Thailand", KASETSART JOURNAL , vol. 41 2007, pages 324-334, XP002688962, Extrait de l'Internet: URL:http://kasetsartjournal.ku.ac.th/kuj_f iles/2008/A0804171228243424.pdf [extrait le 2012-12-10]
- TSUNEHIRO AKI ET AL: "Thraustochytrid as a potential source of carotenoids", JOURNAL OF THE AMERICAN OIL CHEMISTS' SOCIETY, vol. 80, no. 8, 1 août 2003 (2003-08-01), pages 789-794, XP55067749, ISSN: 0003-021X, DOI: 10.1007/s11746-003-0773-2

## Description

L'invention se rapporte à un procédé de culture en mode mixotrophe, notamment en présence d'un éclairement discontinu et/ou variable de lumière, d'un protiste de la classe *Labyrinthulomycete,* en particulier du genre *Schizochytrium.* Le procédé permet d'obtenir un haut rendement en biomasse et un enrichissement des protistes ainsi cultivés en lipides et en caroténoïdes et plus particulièrement en acide docosahexaénoïque (DHA) et en astaxanthine. Le procédé permet ainsi de sélectionner des souches de *Schizochytrium* à caractère mixotrophe, et ayant un haut rendement en lipides et/ou caroténoïdes, et plus particulièrement en acides gras polyinsaturés et en astaxanthine. L'invention se rapporte aussi à une nouvelle souche de protiste appartenant au genre *Schizochytrium,* particulièrement adaptée à la production de lipides et de caroténoïdes. Cette nouvelle souche de *Schizochytrium* est utile pour produire de l'acide docosahexaénoïque (DHA) et de l'astaxanthine en mode mixotrophe.

### Préambule

Les protistes sont des microorganismes à organisation cellulaire simple, c'est-à-dire qu'ils sont généralement unicellulaires et parfois multicellulaires mais sans présenter de tissus spécialisés. Ils peuvent être autotrophes ou hétérotrophes.

Les protistes font l'objet actuellement de nombreux projets industriels car certaines espèces sont capables d'accumuler ou de secréter des quantités importantes de lipides, notamment d'acides gras polyinsaturés.

*Schizochytrium* est un protiste de la famille des *Thraustochytriaceae,* un groupe très répandu de champignon marin. Les Thraustochytrides sont connus pour produire une large gamme de lipides, en particulier des acides gras polyinsaturés, et quelques espèces sont connues pour produire des caroténoïdes (CHATDUMRONG W ET AL, "Optimization of docosahexaenoic acid (DHA) production and improvement of astaxanthin content in a mutant Schizochytrium limacinum isolated from mangrove forest in Thailand", vol. 41, (2007), pages 324 - 334, KASETSART JOURNAL ; TSUNEHIRO AKI ET AL, "Thraustochytrid as a potential source of carotenoids", JOURNAL OF THE AMERICAN OIL CHEMISTS' SOCIETY, (20030801), vol. 80, no. 8, pages 789 - 794).

Parmi les acides gras polyinsaturés, certains acides gras hautement insaturés (AGHI) de la série des oméga-3 (PUFA-ω3), en particulier l'acide éicosapentaénoïque (EPA ou C20:5 ω3) et l'acide docosahexaénoïque (DHA ou C22:6 ω3), et de la série des oméga-6 (PUFA-ω6), en particulier, l'acide arachidonique (ARA ou AA ou encore acide eicosatétraénoïque C20:4 ω6) ont une importance nutritionnelle reconnue et présentent de fortes potentialités en terme d'applications thérapeutiques.

Considéré comme nutriment essentiel, le DHA est nécessaire au développement normal et fonctionnel des cellules, et joue un rôle crucial dans divers processus et fonctions biochimiques. Sa nature polyinsaturée lui confère une importance cruciale vis-à-vis des propriétés de la membrane cellulaire, chez les végétaux comme chez les animaux : fluidité, flexibilité et perméabilité sélective permettant par exemple une adaptation effective, et même la survie, aux basses températures, en particulier chez les poissons.

Le DHA est un constituant structurel majeur du cerveau humain et il en est le principal acide gras. Le DHA représente 15-20 % du cortex cérébral (le cerveau d'un adulte contient au moins 20 g de DHA) et 30-60 % de la rétine. Il est indispensable au développement du système nerveux central et à la fonction rétinienne, par incorporation dans les membranes cellulaires, et joue un rôle capital dans l'acquisition et le maintien satisfaisant des mécanismes de la vision et de la mémoire.

Les huiles de poissons, issues de l'industrie de la pêche, sont actuellement la principale source commerciale de ce type d'acides gras. Toutefois, alors que ces huiles trouvent de nouvelles applications (complément alimentaire en aquaculture, intégration dans les margarines), les ressources halieutiques marines se raréfient du fait d'une activité de pêche intensive.

De nouvelles sources de ces acides gras tels que l'EPA, le DHA et l'ARA doivent donc être recherchées afin de répondre, dans le futur, à la demande croissante du marché pour ce type d'acides gras polyinsaturés.

Outre leur capacité à synthétiser les acides gras *de novo,* les protistes offrent plusieurs avantages par rapport aux huiles de poisson : ils sont cultivables *in vitro* dans des conditions contrôlées, ce qui permet la production d'une biomasse de composition biochimique relativement constante et, d'autre part, contrairement aux huiles de poissons, ils ne présentent pas d'odeur désagréable et leurs lipides ne contiennent pas ou peu de cholestérol.

Enfin, les lipides produits par les protistes ont un profil d'acides gras plus simple que celui des huiles de poissons, ce qui limite les étapes de séparation des acides gras d'intérêt.

Par ailleurs, les caroténoïdes sont également des molécules d'intérêt. Ils sont généralement utilisés en tant que pigments, mais ils ont aussi un rôle important pour la santé de l'homme en tant qu'agents antioxydants. Enfin, ils présentent la capacité de stimuler le système immunitaire.

Pour mettre en oeuvre la production des acides gras et de caroténoïdes par des protistes à l'échelle industrielle, plusieurs facteurs doivent être pris en compte. Par exemple, les cultures peuvent être réalisées en condition autotrophe, mixotrophe ou hétérotrophe selon la souche, la température, les conditions de lumière et la taille des fermenteurs. Par exemple, les cultures peuvent également être réalisées dans des conteneurs d'un litre, dans un laboratoire, dans des photobioréacteurs, et dans des conteneurs de 100 000 litres ou bien dans des bassins ouverts (plusieurs hectares). Toutefois, les dépenses énergétiques et autres ressources telles que la main d'oeuvre et la facilité de poursuivre la culture doivent être prises en compte en développant des conditions de culture idéales. Des bioréacteurs sont notamment décrits dans WO 2009/134111 ou WO 96/21723.

En tout état de cause, il est souhaitable que les protistes soient cultivés dans les conditions optimales pour augmenter le rendement de(s) l'acide(s) gras et du ou des caroténoïde(s) à produire. Ainsi, il est préférable d'avoir un rendement le plus élevé possible (par exemple une biomasse au-delà de 80 g/l de matière sèche, plus de 25 % d'acides gras en poids par rapport au poids total de la matière sèche, et par exemple plus de 0,2 % en poids de caroténoïdes sur le poids total de matière sèche).

L'effet de la lumière sur la croissance n'est pas réservé uniquement aux organismes photosynthétiques (plantes ; cyanobactérie, microalgues et macroalgues). Certains organismes non chlorophylliens possèdent des photorécepteurs qui leur permettent de capter les signaux lumineux essentiels pour leur développement. Les photorécepteurs comme le phytochrome représentent un exemple typique d'un capteur de lumière qui contrôle le développement de l'organisme qui le possède. Différents photorécepteurs ont été décrits à ce jour, à savoir, entre autres, les pigments accessoires, les cryptochromes et phototropines.

*Schizochytrium* est connue pour produire du DHA en mode hétérotrophe ainsi que de l'astaxanthine, lorsqu'il est cultivé en hétérotrophie, en présence d'une lumière fluorescente continue [R. Poontawe, et al. (2008) ; Optimization of DHA and astaxanthin production by Schizochytrium sp. isolated from mangrove forests in Thailand. JSPS-NRCT core university program on development of thermotolerant microbial resources and their applications, pp. 134-135].

Toutefois, dans la perspective d'une exploitation industrielle, un tel mode de culture s'avère inadapté. En effet, pour être rentable, la production de biomasse doit pouvoir être réalisée dans des photo-bioréacteurs fermés de grande dimension. Or, un tel mode de culture est difficile à réaliser, car lorsque la densité des cellules augmente dans le milieu de culture, les cellules ont de plus en plus de difficulté à capter la lumière provenant de l'extérieur du réacteur. Il est alors nécessaire de brasser activement le milieu de culture, ce qui nécessite une importante dépense énergétique.

Il serait souhaitable de pouvoir obtenir des rendements en DHA et astaxanthine plus importants que ce qui est décrit dans l'art antérieur pour une exploitation industrielle plus efficace et rentable.

Pour améliorer le rendement en DHA et en astaxanthine, une alternative à la culture au mode de culture décrit ci-avant serait de pratiquer les cultures en mode mixotrophe, c'est-à-dire avec un apport de lumière de moindre intensité et en présence d'un apport de substrat organique.

Il est entendu que le terme mixotrophe est usuellement employé à l'égard de souches présentant un chloroplaste, capable de se développer en présence de lumière en utilisant deux sources de carbone (organique et inorganique). Dans le cas des souches du genre *Schizochytrium,* un tel chloroplaste n'a pas été identifié. Toutefois, la souche étant à la fois hétérotrophe et réactive à la lumière, le terme mixotrophe sera élargi au sens de l'invention à cette catégorie de souche.

Ainsi, c'est au terme de nombreuses expérimentations dans des conditions de lumière inhabituelles et par l'adjonction de différents substrats que le demandeur est parvenu à isoler des souches de protiste de l'espèce *Schizochytrium,* cultivables en mode mixotrophe permettant, dans les conditions de la présente invention, une production à haut rendement d'acides gras polyinsaturés et de caroténoïdes, notamment de DHA et d'astaxanthine.

Une souche (FCC 1104) représentative des nouvelles souches de *Schizochytrium* ainsi isolées et sélectionnées, a été déposée auprès de la CCAP (Culture Collection of Algae and Protozoa, Scottish Association for Marine Science, Dunstaffnage Marine Laboratory, Oban, Argyll PA371QA, Ecosse, Royaume-Uni) selon les dispositions du Traité de Budapest, sous le numéro d'accession CCAP 4087/1.

Le procédé de culture et de sélection a consisté plus particulièrement à cultiver les protistes en conditions de mixotrophie, en présence d'un éclairement variable et/ou discontinu, notamment sous forme de flashs, avec une gamme de variations d'intensité lumineuse et une fréquence spécifique.

L'alternance rapprochée de phases éclairées et de phases obscures ou de moindre intensité lumineuse, perçue généralement comme stressante par les protistes, a permis, de façon surprenante, d'obtenir des souches de *Schizochytrium* une production élevée de biomasse, de lipides et plus particulièrement d'acides gras polyinsaturés et de caroténoïdes. Cette mise en oeuvre des souches selon l'invention ouvre la perspective d'une production industrielle d'acides gras polyinsaturés, en particulier de DHA, et de caroténoïdes, en particulier d'astaxanthine, dans des fermenteurs bénéficiant d'un apport lumineux réduit, et devrait donc permettre de réaliser des économies d'énergie par rapport aux modes de culture précédemment décrits.

Les différents aspects et avantages de l'invention sont détaillés ci-après.

### Description détaillée

La présente invention a donc pour objet un procédé de culture des protistes de la classe *Labyrinthulomycete,* notamment de la famille *Thraustochytride,* en particulier du genre *Schizochytrium,* en mode mixotrophe dans des conditions d'éclairement discontinu et/ou variable au cours du temps. L'éclairement présente des variations d'intensité, entre les phases éclairées et les phases obscures, dont l'amplitude est comprise entre 30 µmol. m⁻². s⁻¹ et 1000 µmol. m⁻². s⁻¹, de préférence entre 30 et 400 µmol. m⁻². s⁻¹. Ces variations ont lieu entre 2 et 3600 fois par heure, de préférence entre 2 et 200 fois par heure. Ces conditions de culture permettent d'apporter une quantité définie de lumière. Cet apport lumineux peut comporter des phases d'éclairement discontinu et/ou variable, avec des variations d'intensité pouvant avoir des amplitudes identiques ou différentes. L'éclairement peut être notamment sous forme de flashs.

Ce procédé a pour avantage d'augmenter le rendement en biomasse obtenu de la culture. Il a aussi pour avantage d'enrichir les protistes ainsi cultivés en acides gras polyinsaturés, plus particulièrement en acide docosahexaénoïque (DHA), et en caroténoïdes, plus particulièrement en astaxanthine. Ce procédé peut être également utilisé pour sélectionner des souches de la classe *Labyrinthulomycete,* notamment des familles *Thraustochytride* et *Labyrinthulide,* en particulier du genre *Schizochytrium,* à caractère mixotrophe, et ayant un haut rendement en acides gras polyinsaturés, notamment en DHA, et en caroténoïdes, notamment en astaxanthine.

La culture en mode mixotrophe de ce protiste s'effectue préférentiellement en présence de 100 mM à 1,5 M, de préférence de 300 mM à 1,2 M, plus préférentiellement de 500 mM à 1 M, et encore plus préférentiellement de 600 mM à 900 mM d'un substrat carboné organique. L'apport du substrat est assuré continuellement pendant la culture, afin de permettre aux cellules d'accumuler une concentration importante de lipides et de caroténoïdes. Du substrat additionnel est ajouté au milieu de culture pendant le procédé de culture pour maintenir une concentration constante. Ce substrat carboné organique comprend préférentiellement, sous forme pure ou en mélange : du glucose, des dérivés de cellulose, du saccharose et/ou du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des protistes selon l'invention.

Ce procédé est plus particulièrement destiné à la mise en oeuvre de nouvelles souches de la classe *Labyrinthulomycete,* en particulier du genre *Schizochytrium* (Division: Myxomycota, Ordre : Saprolegniales, Famille: Thraustochytriaceae) [ITIS Catalogue of Life, 2010] sélectionnées pour leur caractère mixotrophe, et ayant un haut rendement en acides gras polyinsaturés, notamment en DHA, et en caroténoïdes, notamment en astaxanthine, et notamment pour leur capacité à être cultivées avec un apport lumineux supérieur à 10 µE, dans un milieu riche en éléments organiques, par exemple le milieu Verduyn modifié (sels marins 15 g/L, (NH₄)₂SO₄ 3g/L, KH₂PO₄ 1 g/L, MgSO₄·7H₂0, 0,5 g/L, Na₂EDTA 24 mg/L, ZnSO₄·7H2O 3 mg/L, MnCl₂·2H₂O 3 mg/L, Na₂MoO₄·2H₂O 0,04 mg/L, FeSO₄·7H₂O 10mg/L, pantothenate 3,2mg/L, Thiamine hydrochloride 9,5mg/L, Vitamine B12 0,15mg/L, Antimousse 0,1mL/L) dans lequel est ajouté un substrat carboné organique. De préférence, le substrat carboné organique comprend du glucose, du glycérol, dans une concentration équivalente ou supérieure à 300 mM.

Par « souche », on entend non seulement les souches naturelles du genre *Schizochytrium,* mais également les mutants desdites souches naturelles.

Ces nouvelles souches de *Schizochytrium* peuvent être isolées et sélectionnées selon le procédé de sélection et de culture décrit plus loin.

Une souche représentative des souches de *Schizochytrium* est la souche FCC 1104 isolée par le demandeur et déposée à la CCAP sous le numéro CCAP 4087/1. De telles souches sont capables de produire des quantités significatives de biomasse ainsi que des lipides et des caroténoïdes, et plus particulièrement du DHA et de l'astaxanthine quand elles sont cultivées en mode mixotrophe avec un apport en lumière variable ou discontinu, selon l'invention.

Selon les analyses taxonomiques en cours, la souche CCAP 4087/1 appartient au genre *Schizochytrium.*

Les souches de *Schizochytrium* isolées permettent de produire, en condition de mixotrophie, des quantités significatives de biomasse, ainsi que de lipides et de caroténoïdes, les lipides étant riches en DHA. Ledit DHA peut représenter plus de 40 %, ou plus de 50 %, ou plus de 60 % des lipides totaux contenus dans les protistes, les caroténoïdes étant riches en astaxanthine, et ladite astaxanthine peut représenter plus de 0,1 %, ou plus de 0,15 %, ou plus de 0,2 % en poids sur le poids total de matière sèche.. Les souches peuvent atteindre un niveau de productivité (quantité de produit d'intérêt produit, par litre de culture, par heure) de 0,015 mg/L/h, ou plus de 0,020mg/L/h, ou plus de 0,025 mg/L/h.

Dans la présente invention, les souches de *Schizochytrium* (par exemple, la souche FCC 1104, isolée par le demandeur) qui sont cultivées en conditions mixotrophes en présence d'un éclairement variable et/ou discontinu, notamment sous forme de flashs, permettent de produire de l'astaxanthine. Par contre, en conditions d'hétérotrophie, aucune astaxanthine n'est détectable. De plus, des quantités de biomasse obtenues en mode mixotrophe selon certains mode de réalisation de l'invention sont égales ou même supérieures (par exemple, approximativement 10-18%) aux quantitiés obtenues en conditions d'hétérotrophie. Par, conditions d'hétérotrophie on entend des conditions de culture avec un milieu de culture identique, mais en l'absence de lumière.

L'invention a ainsi pour objet un procédé de culture de protistes souches de *Schizochytrium,* notamment de l'espèce *Schizochytrium* sp. telle que déposée, en mode mixotrophe, en présence d'un éclairement variable ou discontinu au cours du temps, par exemple sous forme de flashs, notamment en vue de produire des acides gras polyinsaturés et des caroténoïdes, tels que le DHA et l'astaxanthine.

Il est apparu qu'un éclairement variable et/ou discontinu des cultures, en particulier dans la mise en oeuvre d'une culture en mode mixotrophe, avait un impact favorable sur le développement des protistes et permettait d'accroître la productivité de ceux-ci, notamment en ce qui concerne leur production de lipides et de caroténoïdes. Sans être lié par la théorie, l'inventeur estime qu'un apport discontinu et/ou variable de lumière aux protistes a pour effet de provoquer un « stress » favorable à la croissance et à la synthèse des lipides, ainsi qu'à la synthèse des caroténoïdes. Ce phénomène pourrait s'expliquer, en partie, par le fait que dans la nature, les protistes ont tendance à accumuler des réserves lipidiques et en caroténoïdes pour résister aux contraintes de leur environnement.

Par éclairement discontinu, il faut entendre un éclairement ponctué par des périodes d'obscurité. Les périodes d'obscurité peuvent occuper plus d'un quart du temps, de préférence la moitié du temps ou plus, durant lequel les algues sont cultivées.

Selon un aspect préféré de l'invention, l'éclairement est discontinu et plus préférentiellement sous forme de flashs. Un flash, au sens de l'invention, est un éclairement lumineux de courte durée, c'est-à-dire de moins de 30 minutes. La durée peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être de 1/10 d'une seconde, ou de 2/10 d'une seconde, ou de 3/10 d'une seconde, ou de 4/10 d'une seconde ou de 5/10 d'une seconde, ou de 6/10 d'une seconde, ou de 7/10 d'une seconde, ou de 8/10 d'une seconde, ou de 9/10 d'une seconde. L'éclairement lumineux, ou le flash, est généralement d'une durée supérieure à 15 secondes. Elle est généralement comprise entre 5 secondes et 10 minutes, de préférence entre 10 secondes et 2 minutes, plus préférentiellement entre 20 secondes et 1 minute.

En général, le nombre de flashs est compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre 2 et 200, préférentiellement entre 10 et 150, plus préférentiellement entre 15 et 100, et plus préférentiellement encore entre 20 et 50 par heure. Les flashs peuvent être émis à intervalle régulier ou non dans le temps. En cas d'émission à intervalle régulier, le nombre de flashs par heure correspond alors à une fréquence (F) ayant une période temporelle (T), étant considéré que F = 1/T. Cette période temporelle peut être comprise entre 1 seconde et 30 minutes, ou entre 1 seconde et 36 secondes, ou encore entre 1,2 seconde et 30 secondes, ou entre 1,44 seconde et 9 secondes, ou entre 1,8 seconde et 6 secondes, ou entre 2,4 secondes et 4,5 secondes. Cette fréquence peut être également comprise entre 18 secondes et 30 minutes, préférentiellement entre 24 secondes et 6 minutes, plus préférentiellement entre 36 secondes et 4 minutes, et plus préférentiellement encore entre 72 secondes et 3 minutes. Le nombre de flashs par heure est choisi en fonction de l'intensité et de la durée des flashs (voir ci-dessous). En général, l'intensité de la lumière apportée sous forme de flashs est entre 30 et 1000 µmol. m⁻². s⁻¹, de préférence entre 30 et 500 µmol. m⁻². s⁻¹, ou 50 et 400 µmol. m⁻². s⁻¹, et plus préférentiellement entre 150 et 300 µmol. m⁻². s⁻¹. 1 µmol. m⁻². s⁻¹ correspond à 1µE m⁻². s⁻¹ (Einstein), unité souvent utilisée dans la littérature.

Selon un mode particulier de l'invention, l'intensité de la lumière est comprise entre 50 et 200 µmol. m⁻². s⁻¹, la période temporelle de la fréquence des flashs est comprise entre 10 secondes et 60 minutes pour une durée de flash comprise entre 1 seconde et 1 minute.

Selon l'invention, quelles que soient les conditions d'éclairement, l'intensité lumineuse apportée aux algues en culture, exprimée en micromoles de photons par seconde par mètre carré (µmol. m⁻². s⁻¹), varie au moins une fois dans une même heure. L'amplitude de cette variation d'intensité de lumière est généralement comprise entre 30 et 1000, ou entre 50 et 800, ou entre 100 et 600 µmol. m⁻². s⁻¹. De préférence, l'amplitude de la variation d'intensité de lumière est entre 70 et 300 µmol. m⁻². s⁻¹ et plus préférentiellement entre 100 et 200 µmol. m⁻². s⁻¹.

Alternativement, en conditions d'éclairement discontinu, ladite intensité lumineuse peut atteindre successivement, plusieurs fois dans l'heure, par exemple, les valeurs 0 et 50 µmol. m⁻². s⁻¹, les valeurs 0 et 100 µmol. m⁻². s⁻¹ ou préférentiellement encore les valeurs 0 et 200 µmol. m⁻². s⁻¹. Elle peut également atteindre successivement, plusieurs fois dans l'heure, par exemple, les valeurs 0 et 300 µmol. m⁻². s⁻¹, les valeurs 0 et 600 µmol. m⁻². s⁻¹, les valeurs 0 et 800 µmol. m⁻². s⁻¹ ou encore les valeurs 0 et 1000 µmol. m⁻². s⁻¹.

Selon un mode de l'invention et quelles que soient les conditions d'éclairement, l'intensité de la lumière apportée à la culture varie en fonction de la densité cellulaire. Plus la culture devient dense, plus la lumière peut être intense. La densité cellulaire est le nombre de cellule par ml et est mesurée selon les techniques connues de l'homme de l'art.

Quand la culture atteint une densité entre 10⁶ et 10⁷ cellules par ml, l'intensité lumineuse peut être augmentée jusqu'à entre 30 et 500 µmol. m⁻². s⁻¹, par exemple, de préférence, entre 50 et 400 µmol. m⁻². s⁻¹. Quand la culture, au stade final, atteint une densité entre 10⁷ et 10⁸ cellules par ml, l'intensité lumineuse peut être augmentée jusqu'à entre 100 et 1000 µmol. m⁻². s⁻¹ par exemple, de préférence, entre 200 et 500 µmol. m⁻². s⁻¹.

Selon un mode de l'invention, la quantité de lumière apportée à la culture dans l'heure reste entre certaines valeurs. Elle est comprise entre environ 2000 et 600 000, de préférence entre 2000 et 300 000 µmol. m⁻². Elle peut être comprise entre environ 4000 et 200 000 µmol. m⁻², par heure.

Selon un autre mode de l'invention, la culture est illuminée avec 120 flashs par heure, chaque flash ayant une durée de 10 secondes et une intensité de 200 µmol. m⁻². s⁻¹, ce qui donne un apport total de lumière par heure de 240 000 µmol. m⁻².

Comme décrit pour l'intensité lumineuse ci-dessus, et selon un mode de l'invention, la quantité de lumière apportée à la culture par heure peut varier en fonction de la densité cellulaire. Au stade initial de la culture quand la densité cellulaire est 10⁵ et 5 x 10⁵ cellules par ml, l'apport total de lumière dans l'heure est généralement compris entre environ 1500 et 8000, de préférence 1500 et 6000 µmol. m⁻², de préférence encore entre 2000 et 5000 µmol. m⁻². Quand la culture atteint une densité entre 10⁶ et 10⁷ cellules par ml, l'apport total de lumière dans l'heure peut être augmenté jusqu'à entre 6000 et 67 000 µmol. m⁻², de préférence, entre 6000 et 50 000 et de préférence encore entre 12 000 et 45 000 µmol. m⁻², par exemple. Au stade final de la culture, à une densité cellulaire entre 10⁷ et 10⁸ cellules par ml, l'apport total de lumière dans l'heure peut être augmenté jusqu'à entre 45 000 et 300 000, par exemple de préférence entre 45 000 et 200 000 µmol. m⁻², et par exemple, de préférence encore, entre 50 000 et 150 000 µmol. m⁻².

Selon un mode de l'invention, par exemple, quand la durée des flashs est par exemple de moins d'une minute, ou de moins d'une seconde, au stade initial de la culture (à une densité cellulaire entre 10⁵ et 5 x 10⁵ cellules par ml), la culture est illuminée avec 30 flashs par heure, chaque flash ayant une durée de 10 secondes et une intensité entre 50 et 100 µmol. m⁻². s⁻¹, ce qui donne un apport total de lumière par heure de 15 000 µmol. m⁻² à 30 000 µmol. m⁻². Puis au stade intermédiaire (à une densité cellulaire entre 10⁶ et 10⁷ cellules par ml), la culture est illuminée avec 50 flashs par heure, chaque flash ayant une durée de 10 secondes et une intensité entre 200 et 300 umol. m⁻². s⁻¹, ce qui donne un apport total de lumière par heure de 100 000 à 150 000 µmol. m⁻². Puis, au stade final de la culture (à une densité cellulaire entre 10⁷ et 10⁸ cellules par ml), la culture est illuminée avec 120 flashs par heure, chaque flash ayant une durée de 10 secondes et une intensité entre 350 et 450 µmol. m⁻². s⁻¹, ce qui donne un apport total de lumière par heure de 420 000 à 540 000 µmol. m⁻².

L'apport de lumière dans les cultures peut être obtenu par des lampes réparties autour de la paroi externe des fermenteurs. Une horloge déclenche ces lampes pour des temps d'éclairement définis. Les fermenteurs se situent préférentiellement dans une enceinte à l'abri de la lumière du jour, dont on peut contrôler la température ambiante.

Ainsi qu'a pu le constater le déposant, le fait que les souches ainsi sélectionnées présentent de bonnes aptitudes à croître en mode mixotrophe, en présence d'une lumière discontinue et/ou variable, prédispose lesdites souches à une production plus élevée d'acides gras polyinsaturés et en caroténoïdes, notamment de DHA et d'astaxanthine.

Le procédé de culture selon l'invention permet ainsi de sélectionner des souches de la classe *Labyrinthulomycete,* notamment des familles *Thraustochytride,* en particulier du genre *Schizochytrium,* à caractère mixotrophe, similaire à celle isolée par le demandeur et déposée à la CCAP sous le numéro CCAP 4087/1, et ayant un haut rendement en acides gras polyinsaturés et en caroténoïdes. Ce procédé de culture est caractérisé en ce qu'il comprend des étapes suivantes :
a) la culture en mode mixotrophe d'une ou plusieurs souches souches de la classe *Labyrinthulomycete,* en particulier, du genre *Schizochytrium* dans des conditions d'éclairement discontinu et/ou variable au cours du temps, l'éclairement présentant des variations d'intensité dont l'amplitude est comprise entre 30 µmol. m⁻². s⁻¹ et 1000, de préférence entre 30 et 400 µmol. m⁻². s⁻¹, ces variations ayant lieu entre 2 et 3600, de préférence 5-400 fois par heure,
b) une étape de maintien de ladite culture sur plusieurs générations, en présence d'un substrat carboné organique dans le milieu de culture, et éventuellement
c) une étape de récupération des protistes ainsi cultivés.

Par étape de récupération, on entend plus particulièrement l'isolement de la ou des souches dont le nombre de cellules s'est accru le plus au cours desdites générations.

Pour réaliser la sélection des souches, différentes souches de la classe *Labyrinthulomycete,* notamment la famille *Thraustochytride,* en particulier, du genre *Schizochytrium,* peuvent être cultivées, en parallèle, sur des microplaques dans une même enceinte, avec un suivi précis des conditions et de l'évolution des différentes cultures. Il est ainsi aisé de connaître la réponse des différentes souches à l'éclairement discontinu et/ou variable et, le cas échéant, à l'adjonction d'un ou plusieurs substrats carbonés organiques dans le milieu de culture. Les souches qui répondent favorablement à l'éclairement discontinu et/ou variable et aux substrats carbonés organiques offrent généralement un meilleur rendement pour la production de caroténoïdes et de lipides sur le plan qualitatif (acides gras polyinsaturés plus abondants dans le profil lipidique et astaxanthine plus abondant parmi les caroténoïdes) et quantitatif (les lipides contiennent une proportion plus élevée de DHA et la matière sèche comporte une proportion plus élevée d'astaxanthine).

Les protistes peuvent être sélectionnés dans un fermenteur à partir d'une population hétérogène et dont on cherche à sélectionner les variants avantagés par le mode de sélection selon l'invention, alliant lumière discontinue et/ou variable, présentant une gamme d'intensité lumineuse et une fréquence spécifiques, avec des conditions de culture mixotrophes. Dans ce cas, la culture est pratiquée en maintenant les protistes en cultures sur de nombreuses générations, puis un isolement des composantes devenues majoritaires dans le milieu de culture est effectué au terme de la culture.

Le procédé de culture selon l'invention permet également de produire des lipides et des caroténoïdes.

Dans ce cas, le procédé selon l'invention comporte en outre les étapes suivantes :
d) une étape de récupération de la matière hydrophobe des protistes, et éventuellement
e) l'extraction du DHA (acide docosahexaénoïque) et de l'astanxanthine de la matière hydrophobe récupérée.

La matière hydrophobe comporte en effet les lipides et les caroténoïdes.

Le procédé de culture selon l'invention peut s'appliquer également à toute espèce du genre *Schizochytrium,* capable de croître dans les conditions mixotrophes selon l'invention, et capable de produire du DHA et de l'astaxanthine.

Le procédé de culture selon l'invention permet d'optimiser la production de la biomasse obtenue de la culture. Il permet également d'enrichir les protistes ainsi cultivés en acides gras polyinsaturés et en caroténoïdes, plus particulièrement en acide docosahexaénoïque (DHA) et en astaxanthine.

L'invention a donc également pour but l'optimisation de la production de biomasse, ainsi que la production de lipides et de caroténoïdes, notamment d'acides gras, via la culture de protistes du genre *Schizochytrium* à caractère mixotrophe, de préférence cultivés ou sélectionnés selon les procédés visés précédemment, puis la récupération des protistes ainsi cultivés pour en extraire le contenu hydrophobe, en particulier les lipides dont le DHA, et les caroténoïdes, dont l'astaxanthine.

Les méthodes d'extraction sélective des lipides, dont le DHA, sont connues de l'homme du métier et sont, par exemple, décrites par [Bligh, E.G. et Dyer, W.J. (1959); A rapid method of total lipid extraction and purification, Can. J. Biochem. Physiol., 37:911-917].

Les méthodes d'extraction et d'analyse des caroténoïdes, dont la lutéine, sont connues de l'homme du métier et sont, par exemple, décrites par Wright et al. (1991) (S.W. Wright, S.W. Jeffrey, R.F.C. Mantoura, C.A. Llewellyn, T. Bjornland, D. Repeta, N. Welschmeyer : Improved HPLC method for the analysis of chlorophylls and carotenoids from marine phytoplankton. Marine ecology progress series : Vol. 77 : 183-196, 1991).

### Exemple 1

Les cultures de *Schizochytrium* sont réalisées dans des fermenteurs (bioréacteurs) de 1 à 2L utiles avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26°C. L'agitation est réalisée grâce à 3 mobiles d'agitation placés sur l'arbre selon la configuration de Rushton (hélices tripales à pompage descendant). La pression d'oxygène dissout est régulée dans le milieu tout au long de la culture, par la vitesse d'agitation (250-600 rpm), le débit d'air (0,25- 1 vvm), voire le débit d'oxygène (0,1-0,5 vvm). Les paramètres de régulation, intégrés dans l'automate de supervision, permettent de maintenir une pO₂ constante à 15%. Le bioréacteur est équipé d'un système luminaire externe entourant la cuve transparente. L'intensité ainsi que les cycles de lumière sont contrôlés par un automate dédié et supervisé par station informatique.

Les réacteurs sont inoculés avec une pré-culture réalisée sur table d'agitation (140 rpm) en enceinte thermostatée (26°C) et éclairée entre 100 et 200 µE. Les pré-cultures et cultures en bioréacteurs sont réalisées dans le milieu Verduyn modifié (sels marins 15 g/L, (NH₄)₂SO₄ 3g/L , KH₂PO₄ 1 g/L, MgSO₄·7H₂0, 0,5 g/L, Na₂EDTA 24 mg/L, ZnSO₄·7H₂O 3 mg/L, MnCl₂·2H₂O 3 mg/L, Na₂MoO₄·2H₂O 0,04 mg/L, FeSO4·7H₂O 10mg/L, pantothenate 3,2 mg/L, Thiamine hydrochloride 9,5mg/L, Vitamine B12 0,15mg/L). Le substrat carboné utilisé pour la culture en mixotrophie classique, en hétérotrophie et en mixotrophie flash (lumière variable/discontinue) en bioréacteur est du glucose à des concentrations entre 300 mM et 1 M.

### Suivi des cultures :

La concentration en biomasse totale est suivie par mesure de la masse sèche (filtration sur filtre GF/F, Whatman, puis séchage en étuve, 105°C, pendant 24h minimum avant pesée).

Concernant la quantification des lipides totaux, 10⁸ cellules/mL ont été extraites. Les méthodes d'extraction des lipides sont connues de l'homme du métier.

Pour la quantification des caroténoïdes et notamment l'astaxanthine 10⁸ cellules/mL ont été extraites. Les méthodes d'extraction et d'analyse des caroténoïdes, dont l'astaxanthine, sont connues de l'homme du métier.

### Eclairement (pour la culture en mixotrophie) :

La culture est illuminée avec 30 flashs par heure, chaque flash ayant une durée de 30 secondes et une intensité de 80 µmol. m⁻². s⁻¹.

L'apport de lumière dans les cultures en bioréacteur a été obtenu par des lampes LED (Diodes Electro Luminescentes) réparties autour de la paroi externe du fermenteur. Une horloge déclenche ces LED pour des temps d'éclairement ou des pulses (pour la culture en mixotrophie flash avec lumière discontinue).

**Résultats (n=3) :**

| | Masse Sèche (g/L) | Lipides totaux (% de la MS) | % DHA | Astaxanthine (mg/g de MS) |
|---|---|---|---|---|
| Mixotrophie Flash | 140 +/- 2,1 | 28 +/- 0,6 | 43 +/- 1,8 | 2 +/- 0,1 |
| Hétérotrophie | 118,1 +/- 2,5 | 28,8 +/- 0,6 | 45,2 +/- 2 | 0 |

## Revendications

1. Procédé comprenant l'étape suivante:
a) la culture en mode mixotrophe d'une ou plusieurs souches du genre *Schizochytrium* dans des conditions d'éclairement discontinu et/ou variable au cours du temps, l'éclairement présentant des variations d'intensité, entre les phases éclairées et les phases obscures, dont l'amplitude est comprise entre 30 µmol. m⁻². s⁻¹ et 1000 µmol. m⁻². s⁻¹, ces variations ayant lieu entre 2 et 3600 fois par heure.

2. Procédé selon la revendication 1, **caractérisé en ce que** l'éclairement présente des variations d'intensité dont l'amplitude est comprise entre 30 µmol. m⁻². s⁻¹ et 400 µmol. m⁻². s⁻¹, ces variations ayant lieu entre 2 et 200 fois par heure.

3. Procédé selon la revendication 1 ou 2, **caractérisé en ce que** la culture s'effectue en présence d'un substrat carboné organique à une concentration de 100 mM à 1,5 M, de préférence de 300 mM à 1,2 M, plus préférentiellement de 500 mM à 1 M, et encore plus préférentiellement de 600 mM à 900 mM, le substrat carboné organique étant choisi parmi le saccharose, le glycérol, le glucose et les dérivés de cellulose et un mélange de ces molécules.

4. Procédé selon la revendication 3, **caractérisé en ce que** ledit substrat carboné organique présent dans le milieu de culture comprend au moins 300 mM de glucose et/ou glycérol.

5. Procédé selon l'une quelconque des revendications 1-4, **caractérisé en ce que** l'amplitude des variations d'intensité est comprise entre 30 et 1000 µmol. m⁻². s⁻¹ de préférence entre 70 et 300 µmol. m⁻². s⁻¹, et plus préférentiellement entre 100 et 200 µmol. m⁻². s⁻¹.

6. Procédé selon l'une quelconque des revendications 1 à 5, **caractérisé en ce que** l'apport de lumière s'effectue sous forme de flashs.

7. Procédé selon la revendication 6, **caractérisé en ce qu'**un flash a une durée comprise entre 1/10 seconde et 10 minutes, de préférence 5 secondes et 10 minutes, de préférence encore entre 10 secondes et 2 minutes, plus préférentiellement entre 20 secondes et 1 minute.

8. Procédé selon la revendication 6 ou la revendication 7, **caractérisé en ce que** le nombre de flashs est entre 5 et 3600, de préférence entre 10 et 150, préférentiellement entre 15 et 100, et plus préférentiellement entre 20 et 50 fois par heure.

9. Procédé selon l'une quelconque des revendications 1 à 6, **caractérisé en ce que** l'apport total de lumière par heure en micromoles de photons est entre 2000 à 600 000, de préférence entre 2000 à 200 000 µmol. m⁻².

10. Procédé selon l'une quelconque des revendications 1 à 9, **caractérisé en ce qu'**il comprend en outre les étapes suivantes:
b) une étape de maintien de ladite culture sur plusieurs générations en présence d'un substrat carboné organique dans le milieu de culture, et éventuellement
c) une étape de récupération des protistes ainsi cultivés, et éventuellement
d) une étape de récupération de la matière hydrophobe, et éventuellement
e) l'extraction du DHA (acide docoahexaénoïque) et de l'astaxanthine de la matière hydrophobe récupérée.

11. Procédé selon l'une quelconque des revendications 1 à 10, **caractérisé en ce que** ledit protiste du genre *Schizochytrium* correspond à la souche FCC 1104, déposée auprès de la CCAP (Culture Collection of Algae and Protozoa), sous le numéro CCAP 4087/1.

## Patentansprüche

1. Verfahren, umfassend den folgenden Schritt:
a) die Kultur im mixotrophen Modus eines oder mehrerer Stämme der Gattung *Schizochytrium* unter Bedingungen der im Zeitverlauf diskontinuierlichen und/oder variablen Beleuchtung, wobei die Beleuchtung Stärkeschwankungen zwischen hellen Phasen und dunklen Phasen aufweist, deren Amplitude zwischen 30 µmol ·m⁻² ·s⁻¹ und 1000 µmol ·m⁻² ·s⁻¹ inklusive beträgt, wobei diese Schwankungen zwischen 2 und 3600 Mal pro Stunde stattfinden.

2. Verfahren nach Anspruch 1, **dadurch gekennzeichnet, dass** die Beleuchtung Stärkeschwankungen aufweist, deren Amplitude zwischen 30 µmol ·m⁻² ·s⁻¹ und 400 µmol ·m⁻² ·s⁻¹ inklusive beträgt, wobei diese Schwankungen zwischen 2 und 200 Mal pro Stunde stattfinden.

3. Verfahren nach Anspruch 1 oder 2, **dadurch gekennzeichnet, dass** die Kultur bei Anwesenheit eines organischen kohlenstoffhaltigen Substrats in einer Konzentration von 100 mM bis 1,5 M, vorzugsweise von 300 mM bis 1,2 M, vorzugsweiser von 500 mM bis 1 M und noch vorzugsweiser von 600 mM bis 900 mM stattfindet, wobei das organische kohlenstoffhaltige Substrat aus der Saccharose, dem Glycerol, der Glucose und den Zellulosederivaten und einem Gemisch dieser Moleküle ausgewählt ist.

4. Verfahren nach Anspruch 3, **dadurch gekennzeichnet, dass** das in dem Kulturmilieu enthaltene organische kohlenstoffhaltige Substrat mindestens 300 mM Glucose und/oder Glycerol umfasst.

5. Verfahren nach einem der Ansprüche 1-4, **dadurch gekennzeichnet, dass** die Amplitude der Stärkeschwankungen zwischen 30 und 1000 µmol ·m⁻² ·s⁻¹, vorzugsweise zwischen 70 und 300 µmol ·m⁻² ·s⁻¹ und vorzugsweiser zwischen 100 und 200 µmol ·m⁻² ·s⁻¹ inklusive beträgt.

6. Verfahren nach einem der Ansprüche 1 bis 5, **dadurch gekennzeichnet, dass** die Zufuhr von Licht in Form von Blitzen erfolgt.

7. Verfahren nach Anspruch 6, **dadurch gekennzeichnet, dass** ein Blitz eine Dauer zwischen 1/10 Sekunde und 10 Minuten, vorzugsweise 5 Sekunden und 10 Minuten, vorzugsweise auch zwischen 10 Sekunden und 2 Minuten, noch vorzugsweiser zwischen 20 Sekunden und 1 Minute hat.

8. Verfahren nach Anspruch 6 oder Anspruch 7, **dadurch gekennzeichnet, dass** die Anzahl der Blitze zwischen 5 und 3600, vorzugsweise zwischen 10 und 150, in bevorzugter Weise zwischen 15 und 100 und vorzugsweiser zwischen 20 und 50 Mal pro Stunde beträgt.

9. Verfahren nach einem der Ansprüche 1 bis 6, **dadurch gekennzeichnet, dass** die Gesamtzufuhr von Licht pro Stunde in Mikromol Photonen zwischen 2000 bis 600000, vorzugsweise zwischen 2000 bis 200000 µmol ·m⁻², beträgt.

10. Verfahren nach einem der Ansprüche 1 bis 9, **dadurch gekennzeichnet, dass** es ferner die folgenden Schritte umfasst:
b) einen Schritt des Haltens der Kultur über mehrere Generationen eines organischen kohlenstoffhaltigen Substrats in dem Kulturmilieu und eventuell
c) einen Schritt der Rückgewinnung der derart kultivierten Protisten und eventuell
d) einen Schritt der Rückgewinnung des hydrophoben Materials und eventuell
e) die Extraktion der DHA (Docosahexaensäure) und des Astaxanthins aus dem zurückgewonnenen hydrophoben Material.

11. Verfahren nach einem der Ansprüche 1 bis 10, **dadurch gekennzeichnet, dass** der Protist der Gattung *Schizochytrium* dem Stamm FCC 1104 entspricht, hinterlegt bei der CCAP (Culture Collection of Algae and Protozoa) unter der Nummer CCAP 4087/1.

## Claims

1. A method comprising the following step:
a) culture, in mixotrophic mode, of one or more strains of the genus *Schizochytrium* under conditions of illumination that is discontinuous and/or variable over time, the illumination having variations in intensity, between the illuminated phases and the phases of darkness, the amplitude of which is comprised between 30 µmol ·m⁻² ·s⁻¹ and 1,000 µmol ·m⁻² ·s⁻¹, these variations taking place between 2 and 3,600 times per hour.

2. The method according to claim 1, **characterized in that** the illumination has variations in intensity, the amplitude of which is comprised between 30 µmol ·m⁻² ·s⁻¹ and 400 µmol ·m⁻² ·s⁻¹, these variations taking place between 2 and 200 times per hour.

3. The method according to claim 1 or 2, **characterized in that** the culture is carried out in the presence of an organic carbon-containing substrate at a concentration from 100 mM to 1.5 M, preferably from 300 mM to 1.2 M, more preferentially from 500 mM to 1 M, and even more preferentially from 600 mM to 900 mM, the organic carbon-containing substrate being selected from sucrose, glycerol, glucose and cellulose derivatives and a mixture of these molecules.

4. The method according to claim 3, **characterized in that** said organic carbon-containing substrate present in the culture medium comprises at least 300 mM of glucose and/or glycerol.

5. The method according to any one of claims 1 to 4, **characterized in that** the amplitude of the variations in intensity is comprised between 30 and 1,000 µmol ·m⁻² ·s⁻¹, preferably between 70 and 300 µmol ·m⁻² ·s⁻¹, and more preferentially between 100 and 200 µmol ·m⁻² ·s⁻¹.

6. The method according to any one of claims 1 to 5, **characterized in that** the light supply is in the form of flashes.

7. The method according to claim 6, **characterized in that** a flash has a duration comprised between 1/10 second and 10 minutes, preferably 5 seconds and 10 minutes, more preferably between 10 seconds and 2 minutes, more preferentially between 20 seconds and 1 minute.

8. The method according to claim 6 or claim 7, **characterized in that** the number of flashes is between 5 and 3,600, preferably between 10 and 150, preferably between 15 and 100, and more preferentially between 20 and 50 times per hour.

9. The method according to any one of claims 1 to 6, **characterized in that** the total light supply per hour in micromoles of photons is between 2,000 to 600,000, preferably between 2,000 to 200,000 µmol ·m⁻².

10. The method according to any one of claims 1 to 9, **characterized in that** it further comprises the following steps:
b) a step of maintaining said culture over several generations in the presence of an organic carbon-containing substrate in the culture medium, and optionally
c) a step of recovery of the thus cultured protists, and optionally
d) a step of recovery of the hydrophobic matter, and optionally
e) the extraction of DHA (docosahexaenoic acid) and astaxanthin from the hydrophobic matter recovered.

11. The method according to any one of claims 1 to 10, **characterized in that** said protist of the genus *Schizochytrium* corresponds to the strain FCC 1104, deposited at the CCAP (Culture Collection of Algae and Protozoa), under number CCAP 4087/1.
